# EUROPEAN PATENT SPECIFICATION

(11) **EP 1 377 280 B1**
(45) Date of publication and mention of the grant of the patent: **18.07.2007**
(21) Application number: 02725535.5
(22) Date of filing: 05.04.2002
(51) Int. Cl.: A23L 1/236, A23L 1/308, A23L 1/29, A61P 3/04, A61K 31/716, A23C 9/13, A23L 1/09

(54) **USE OF BULKING AGENTS AS SATIETY AGENTS**
VERWENDUNG VON FÜLLSTOFFE ALS SÄTTIGUNGSMITTEL
UTILISATION D'AGENTS GONFLANTS COMME AGENTS DE SATIÉTÉ

(30) Priority: 09.04.2001 US 282866 P
(43) Date of publication of application: 07.01.2004
(73) Proprietor: Danisco USA, Inc., Ardsley, NY 10502-2605 (US)
(72) Inventor: CRAIG, Stuart, Sommers, NY 10589 (US); OLINGER, Philip, M., Reno, NV 89511 (US); PEPPER, Tammy, Weybridge, Surrey KT13 9JZ (GB)
(74) Representative: Hjelt, Dag Silvio Hjalmar Andrea
(86) International application number: PCT/US2002/010749
(87) International publication number: WO 2002/080850

(56) References cited:
- EP-A- 0 749 697
- WO-A-01/08505
- WO-A-01/76394
- DE-U- 29 915 656
- US-A- 5 098 730
- US-A- 5 753 253
- SHAFER R B ET AL: "EFFECTS OF XYLITOL ON GASTRIC EMPTYING AND FOOD INTAKE" AMERICAN JOURNAL OF CLINICAL NUTRITION, vol. 45, no. 4, 1987, pages 744-747, XP000903649 ISSN: 0002-9165

## Description

### FIELD OF THE INVENTION

This invention relates to the use of polydextrose and other sugar polymers for controlling appetite in humans.

### BACKGROUND OF THE INVENTION

There are approximately 34 million Americans at least twenty percent above their desired weights. Many of them attribute their obesity to overeating, and/or being unable to control their appetite.

The obesity caused by excessively high caloric intake and accumulation of surplus fat is a health risk and often leads to various types of degenerative diseases. For example, obesity is a contributory factor to the increased incidence of cardiovascular disease, hypertension, hypercholesterolemia, non-insulin-dependent diabetes, and various cancers, including uterus, breast, gallbladder, colon, rectum and prostate.

In addition, obesity has a negative weight related impact on mortality, such that in extreme or morbid obesity, the mortality rate may be 1200 percent above normal.

Dieting, bariatrics and cytotherapy are of major concern to patients who suffer from obesity-caused diseases and also to healthy people, who, for cosmetic reasons, wish to control their caloric intake and thereby decrease their weight.

Dieting often requires that significant limitations be placed on the amount of caloric intake, and the amount of fat and carbohydrates consumed by an individual are invariably diminished in a successful dietary plan. However, due to the inherent causes of obesity and overeating, dieting by itself is often unsuccessful in achieving the patients' goals. There are several primary reasons for this. First, there is an immense amount of patience and will-power required by the dieter to lose significant amounts of weight. Second, and perhaps more important, dieting is seldom satisfactory, since the dieter is often hungry, and wishes to ingest high-calorie foods or other foods which he misses and which he cannot ingest when placed on the diet. Third, are the inherent reasons that people eat to excess. For example, it is well known that the vast majority of over-eating is done to satisfy anxiety. Associated with the anxiety is the feeling of an empty stomach and an insatiable appetite for food. Thus, caloric intake often is not engaged in for the sole purpose of satisfying hunger and meeting metabolic needs, but also to satisfy secondary needs in the individuals life.

Although many attempts have been made to cause one to feel "full" or satiated when the stomach is not in fact filled with food, each solution to date has its own problems. For example, some people have attempted semi-starvation diets. These are universally effective in short term weight loss, but those subjecting themselves to these diets often regain weight after resumption of less restricted diets. Moreover, long term use of semi-starvation diets is nutritionally unsound because of the development of multiple deficiencies of essential nutrients.

Surgical devices have also been employed to control appetite. Intragastric balloons, for example, have been placed endoscopically according to the theory that they increase the amount of gastric distension and thus augment satiety responses. However, they have been discontinued because, while they were not shown to be any better than restricted diets in promoting weight loss, their long term use was associated with severe side effects, such as gastric ulceration and migration of the balloons into the small intestine, resulting in intestinal obstructions.

Another approach has been the use of chemical appetite suppressants. These include weight control agents which act on the central nervous system to suppress appetite. For example, one major subclass of central nervous system appetite suppressant drugs interacts with cathecolaminergic receptors in the brainstem. Examples include amphetamine, phenmetrazine, manizidol, diethylproprion and phenylpropanolamine. Unfortunately, each of these agents have potential for addiction and, at doses which effectively reduce appetite, i.e., suppress food intake by 20-30%, they induce significant central nervous system side effects, such as nervousness, loss of concentration and insomnia.

Another type of central nervous system appetite control drugs interferes with serotonergic systems. For example, D-fenfluramine releases and depletes brain serotonin, but it causes sedation at appetite suppressant levels and may precipitate depression upon its withdrawal. Another agent, fluoxetine, an inhibitor of serotonin re-uptake in the brainstem, often cause nausea and asthma (weakness and/or lassitude) at effective appetite control doses.

Another type of weight control agents are drugs which promote malabsorption of nutrients through suppression of digestive enzymes. Examples include Acarbose, a bacterial inhibitor of amylase and brush-border glycosidases; and tetrahydrolipostatin, a fungal inhibitor of lipases. These agents work by preventing digestion of carbohydrates and/or fats, thus creating an effective reduction in the number of calories absorbed, despite continued consumption. One drawback, however, of the use of these drugs is that virtually complete inhibition of the respective enzymes must be maintained throughout the digestive period, a situation that can be rarely achieved. Thus, Acarbose was shown to be ineffective in humans, and tetrahydrolipostatin reduced human absorption of fat by only 30%. A second major drawback to this approach is that subjects taking these agents develop hyperphagia for other foodstuffs. For example, subjects taking tetrahydrolipostatin will consume more carbohydrate to compensate for the loss of fat absorption. Thus, the loss of calories from malabsorption is compensated by an increased intake of food, especially of foodstuffs of a different class.

A third class of weight control agents includes non-caloric non-nutritive dietary substitutes, like saccharin, dipeptide sweeteners, such as aspartame and the like, and sucrose polyester, a fat substitute. These agents, while not absorbed, provide a taste and/or texture like the nutrient for which they are substituted. The disadvantage of these substitutes is that persons develop a hyperphagia to compensate for the reduction of calories by the substitution.

What has been needed, heretofore, but has not been achievable is a low calorie material which suppresses appetite and causes one to feel satiated, without causing undesirable side effects.

Xylitol is an example. It is a pentose sugar alcohol and it has been shown to be a potentially important agent in dietary control. Shafer, et al. as described in Am. J. Clin. Nutr., 1987, 45:744-747, studied the effects of xylitol on gastric emptying of the solid-food component of a complex meal. After ingestion of 25 g xylitol, gastric emptying was markedly prolonged (T-1/2: 58 ± 5 min. control, vs. 91 ± 7 minutes after xylitol intake). They also showed that food intake after oral preloading of 25 g xylitol with water led to intake of 690 ± 45 kcal, as compared to 920 ± 60 Kcal for control, a 25% reduction in calories.

The present inventors have found that, inter alia, polydextrose or hydrogenated polydextrose alone or in combination can be used in controlling the appetite in animals, especially mammals, and avoids the disadvantages and side effects associated with satiety agents used heretofore. The present inventors have also found that polydextrose, including hydrogenated polydextrose, or combination thereof acts synergistically with xylitol and if administered to an animal in synergistic effective amounts can also be used as an appetite suppressant.

### SUMMARY OF THE PRESENT INVENTION

The present invention is set out in the appended claims.

### BRIEF DESCRIPTION TO THE DRAWINGS

Figure 1 depicts graphically the mean test meal energy intake for days 1 and 10 in the 10 day study induced by the yoghurts containing either h-polydextrose, xylitol or 1:1 weight ratio of xylitol:h-polydextrose, as compared with a control.
Figure 2 depicts graphically the mean combined (pre-load and test lunch) energy intakes by the test subjects after consumption of the aforesaid yoghurts.
Figure 3 depicts graphically the mean suppression of hunger immediately following consumption of 3 separate yoghurts containing either h-polydextrose, xylitol or a 1:1 weight ratio of Xylitol: h-polydextrose for the test subjects.
Figure 4 depicts graphically the mean relative suppression of hunger immediately following consumption of the aforesaid yoghurts for test subjects.
Figure 5 depicts graphically the mean increase in fullness on days 1 and 10 in the 10 day study induced by the aforesaid yoghurt.
Figure 6 depicts graphically the mean relative increase in fullness induced by the aforesaid yoghurts.

### DETAILED DESCRIPTION OF THE INVENTION

In an embodiment, the present invention is directed to the use of a food satiety agent to control an animal's appetite and food intake and to provide a feeling of fullness resulting from its ingestion. In an embodiment of the present invention, the food satiety agent is polydextrose, including hydrogenated polydextrose or mixture thereof while in another embodiment, the food satiety agent is a sugar polymer, including hydrogenated sugar polymer as defined herein, or mixture thereof. In another embodiment of the present invention, the satiety agent, e.g., sugar polymer or hydrogenated sugar polymer, including a mixture thereof in combination with a sugar alcohol, e.g., xylitol is administered in synergistic effective amounts to reduce food intake and/or reduce the appetite of said animal.

As used herein, the term "food satiety agent" or "satiety agent" refers to a sugar polymer, including hydrogenated sugar polymer or combination thereof, e.g., polydextrose, hydrogenated polydextrose or mixture thereof.

Moreover, as used herein, the term "polydextrose" is a low calorie polymer of glucose that is resistant to digestion by the enzymes in the stomach. It includes polymer products of glucose which are prepared from glucose, maltose, oligomers of glucose or hydrolyzates of starch, which are polymerized by heat treatment in a polycondensation reaction in the presence of an acid, e.g., Lewis acid, inorganic acid or organic acid, including monocarboxylic acid, dicarboxylic acid, and polycarboxylic acid, such as, but not limited to the products prepared by the processes described in U.S. Patent Nos. 2,436,967, 2,719,179, 4,965,354, 3,766,165, 5,051,500, 5,424,418, 5,378,491, 5,645,647 or 5,773,604. The term "polydextrose" also includes those polymer products of glucose prepared by the polycondensation of glucose, maltose, oligomers of glucose, or starch hydrolyzates described hereinabove in the presence of an acid, but in addition in the presence of a sugar alcohol, e.g., polyol, such as in the reactions described in U.S. Patent Nos. 3,766,165. Moreover, the term polydextrose includes the glucose polymers which have been purified by techniques described in the art, including any and all of the following but not limited to (a) neutralization of any acid associated therewith by base addition thereto, or by passing a concentrated aqueous solution of the polydextrose through an adsorbent resin, a weakly basic ion exchange resin, a type II strongly basic ionexchange resin, mixed bed resin comprising a basic ion exchange resin or a cation exchange resin, as described in U.S. Patent Nos. 5,667,593 and 5,645,647, or (b) decolorizing by contacting the polydextrose with activated carbon or charcoal, by slurrying or by passing the solution through a bed of solid adsorbent or by bleaching with sodium chlorite, hydrogen peroxide and the like; (c) molecular sieving methods, like UF, RO (reverse osmosis), size exclusion, and the like; (d) or enzymically treated polydextrose or (e) any other art recognized techniques known in the art.

Moreover, the term "polydextrose" includes hydrogenated polydextrose which, as used herein, includes hydrogenated or reduced polyglucose products prepared by techniques known to one of ordinary skill in the art. Some of these techniques are described in U.S. Patent No. 5,601,863 to Borden, et al., 5,620,871 to Caboche and 5,424,418, to Duflot.

The term "sugar polymer", as used herein refers to a food acceptable non-toxic polymer of a sugar which is resistant to enzyme digestion in the human stomach. It may be a natural product, or a synthetic product prepared from the polymerization of a sugar. It includes products prepared by any of the processes described hereinabove for polydextrose, however, one or more sugars may be utilized as the starting material. The term "sugar polymer" includes polydextrose, but also includes other food acceptable products in which sugars are used in lieu of glucose in the polycondensation reaction, as described hereinabove. Thus, it includes the products from the polymerization of sugars in the presence of acid and optionally, but preferably in the presence of sugar alcohol, as well as the purified products thereof, including utilizing any of the purified techniques described hereinabove. It also includes hydrogenated sugar polymers which refers to sugar polymers, as defined herein, which have been reduced or hydrogenated by techniques known in the art, such as those described by the aforementioned U.S. Patent Nos. 5,601,863, 5,620,871 and 5,424,418

By "sugars", as used herein, it is meant monosaccharides, disaccharides or oligosaccharides. Although the D and L sugars may be utilized, it is preferred that the sugars have the D configuration.

As used herein, the monosaccharides contain from 3-6 carbon atoms and include aldoses e.g., hexoses. Examples of monosaccharides include glyceraldehydes, erythrose, threose, ribose, arabinose, xylose, lyxose, allose, altrose, glucose, mannose, idose, galactose, talose, erythrulose, ribulose, xylulose, psicose, fructose, sorbose, tagatose, and the like. The monosaccharides may exist as either the D or L isomers, although the D-isomer is preferred.

Examples of disaccharides include maltose, lactose, sucrose, trehalose, isomaltose and isomaltulose and the like.

The oligosaccharides contain 3-10 sugar units, and more preferably 3-6 sugar units. Examples of oligosaccharides include Fructo-oligosaccharides, maltotriose and the like.

However, it is preferred that the sugars utilized in the polymerization reaction are pentose or hexoses.

Examples of sugar polymers include galacto-oligosaccharides, pyrodextrin (resistant maltodextrin), trademarked as Fibersol^{®} or Pine Fiber^{®}, inulin, hydrolyzed guar gum, trademarked as Benefiber^{®}.

In a preferred embodiment, the satiety agent is polydextrose, including hydrogenated polydextrose.

Administration of the satiety agent to the animal either alone or in combination with a sugar alcohol, e.g., xylitol, in synergistic effective amounts also controls the appetite and/or reduces the food intake of said animal, e.g., mammal at a meal or at snack time. This synergistic effect for controlling hunger and providing the fullness sensation experienced by mammals, e.g., humans, after ingestion thereof is greater than exhibited when either the polyol or the satiety agent were administered by themselves.

It is preferred that the satiety agent is substantially pure. It may be made substantially pure using conventional techniques known to one of the ordinary skill in the art such as, chromatography, including column chromatography, HPLC, and the like.

It is more preferred that the satiety agent is at least about 80% pure, i.e., at least about 80% of the impuritios are removed. More preferably, the satiety agent is at least 85% pure and even more preferably, it is at least 90% pure.

The satiety agent, either alone or in synergistic effective amount with a polyol, is administered to the subject in food a intake suppressing effective amounts to effect appetite suppression. As used herein, the term subjects refers to animals, especially mammals. Preferred mammals include but are not limited to dogs, cats, horses, pigs, cows, sheep and man. The most preferred mammal is man.

As used herein, the term "food intake suppressing effective amounts" or synonym thereto refers to the amount of satiety agent, e.g., polydextrose, either alone or in synergistic effective amounts with polyol on a dry weight basis per Kg of body weight which is to be administered to suppress food intake. It is within the purview of one of ordinary skill in the art to calculate such amounts considering the method of administration. It is preferred that the satiety agent is administered in amounts ranging from about 15 to about 700 mg/kg/day and more preferably from about 200 to about 450 mg/kg/day. Thus, it is preferred that the satiety agent is administered in amounts ranging from about 1 g to about 50 g per day and more preferably ranging from about 15 to about 30 g per day, for animals, e.g., humans.

The timing of the administration of the satiety agent, either alone or in synergistic effective amounts with a polyol, of the present invention is not critical and can be taken based upon individual needs. For example, the satiety agent, either alone or in synergistic effective amounts with a polyol can be taken when a feeling of hunger occurs on a schedule, such as at least once per day or at least twice per day. However, it may be beneficial to administer the satiety agent, either alone or in synergistic effective amounts with a polyol based upon the timing of meals. For example, the satiety agent, either alone or in synergistic effective amounts with a polyol may be administered prior to or at one, two or three meals or snacks per day or prior to each meal or snack if more than 3 meals or snacks are eaten each day. By taking the satiety agent, either alone or in synergistic effective amounts with a polyol before a meal or snack for a sufficient time for the satiety agent to be effective in suppressing hunger and/or inducing fullness, the animal, e.g., mammal, will be ingesting less food between meals and/or during meals. It is preferred that the satiety agent of the present invention be administered to the animal, e.g., mammal, from about one-quarter of an hour to about twelve hours, and more preferably up to about four hours and most preferably up to about 1 ½ hours before a meal or snack. It is more preferred that the satiety agent is administered from about one half hour to about four hours and more preferably from about one half hour to about 1½ hours before a meal or snack. The appetite is sufficiently depressed that a smaller than normal quantity of food is ingested, as the satiety agent, either alone or in synergistic effective amounts with a polyol of the present invention will act to curb the appetite. Typically, then the satiety agent, either alone or in synergistic effective amounts with a polyol of the present invention will be taken sometime in a period prior to a meal or at the time a usual meal is eaten, and this will serve to decrease the intake of food at the meal or may even eliminate the meal altogether, as the satiety agent, e.g., polydextrose, either alone or in synergistic effective amounts with a polyol may provide a sufficient feeling of satiation to eliminate some normally eaten meals or snacks.

The satiety agent, either alone or in synergistic effective amounts with a polyol may be administered in any form suitable for oral administration.

The satiety agent, either alone or in synergistic effective amounts with a polyol may be administered preferably in the form of a powder or in aqueous solution and is to be ingested in the aforesaid amounts. The dosage administered may contain only satiety agent, either alone or in synergistic effective amounts with a polyol or it may contain, in addition, auxiliaries used in the food arts, such as flavoring agents, colorants, preservatives, taste masking agents, sweeteners, especially high potency sweeteners and the like. In one embodiment of the present invention, the satiety agent, either alone or in synergistic effective amounts with a polyol may be mixed with any one or more of the aforesaid auxiliaries. For example, the satiety agent may be administered with a high potency sweetener. Examples of high potency sweeteners include aspartame, cyclamates, saccharin, acesulfame, neohesperidin dihydrochalcone, sucralose, alitame, stevia sweeteners, glycyrrhisin, thaumatin and the like and mixtures thereof. The preferred high potency sweetener is a dipeptide sweetener, e.g., aspartame.

The satiety agent, either alone or in synergistic effective amounts with a polyol may be added to a food which is ingested by the subject at or before the meal, in accordance with the present invention, in food intake suppressing effective amounts. Such foods include, but are not limited to, yoghurt, butters, including fruit butters, cream cheese, jellies, jam, preserves, marmalades, cereal, granola bars, confectionery, crackers, dairy desserts, e.g., mousse, frozen foods such as ice cream, sherbet and water ices, baked goods, such as cakes, cookies, pastries and other foodstuffs; in beverages, such as soft drinks, aqueous solutions, including water, milk and the like; syrups, toppings, sauces, and puddings, in salad dressings, mayonnaise, gravy mix, barbecue sauce or other sauce used with meat, fish or poultry, sauces used for pasta and other foods. However, since the objective is to reduce the caloric intake, it is preferred that if added to a food, the food that it is added to is low in calories, such as yoghurt, milk, water, diet drinks, cereal, and granola bars.

The satiety agent, either alone or in synergistic effective amounts with a polyol may also be administered to the mammal with adjuvants which in the amounts added do not materially affect the appetite suppression of the mammal. Examples include polyalcohols (hereinafter referred to as polyols), such as xylitol, sorbitol, maltitol, mannitol, isomalt, and the like which are present in substantially non-appetite suppressing effective amounts; with hydrocolloids, sugars or sugar derivatives, such as trehalose; pectin; cyclodextrin; pyrodextrin ("resistant maltodextrin"), trademarked as Fibersol^{®} or Pine Fiber^{®}; inulin; hydrolyzed guar gum; trademarked as Benefiber^{®}; fructooligosaccharide; galactooligosaccahrides, hydrogenated starch hydrolyzates and the like. In one embodiment of the present invention, if the additional adjuvant has appetite suppressant activity, and if it is administered with the satiety agent of the present invention, the additional appetite suppressant is present in amounts so that its presence does not substantially affect the appetite suppressing activity of the satiety agent, i.e., in this embodiment, the appetite suppressant activity is primarily due to the satiety agent, as defined herein. In other words, the additional adjuvant is present in minor amounts, while the satiety agent is present in major amounts. If an adjuvant is present, in one embodiment of the present invention, it is preferably present in an amount within the range of from about 0.0001% by weight to an amount less than about 50% by weight of the satiety agent and more preferably in an amount within the range of about 0.001% to about 10% by weight. Thus, for example, xylitol may be present in a food or composition along with the satiety agent; and this food or composition may be given to the animal. However, in this embodiment, if present, the xylitol is present in amounts that will not substantially affect the appetite of the animal. Moreover, in this embodiment, it is not present in synergistic effective amounts, as defined herein.

Without wishing to be bound, it is believed that the satiety agents, e.g., polydextrose, either alone or in synergistic effective amounts with a polyol function to delay gastric emptying. This delay in gastric emptying may be attributed to the slow and incomplete absorption of the satiety agent, e.g., polydextrose, either alone or in synergistic effective amounts with a polyol which results in luminal osmolality and delayed emptying.

As used herein, the term h-polydextrose or PDXh mean the same thing and as a shorthand notation for hydrogenated polydextrose.

The following non-limiting examples further illustrate the invention.

### EXAMPLE 1

Eight female and seven male subjects with mean ages ranging from about 29.5 to 30.7 yr. were used in the study. They were lean (having BMI of 21.6 Kg/m²-23.8 Kg/m²), and they exercised between 2-3 times per week.

There were four experimental conditions that all subjects completed in a counterbalanced order with each condition separated by a one-week washout period. During each experimental condition, subjects were provided with either control or test yoghurts to consume as part of their normal diet for 10 consecutive days. The four experimental conditions differed according to the type of carbohydrate added to the yoghurt consumed:

| | |
|---|---|
| Cont (Control) - | 25 g/day of sucrose |
| Xyl (Xylitol) - | 25g/day of Xylitol |
| XylPDXh (Xylitol Polydextrose) Mixture- | 12.5g/day of h-polydextrose and 12.5g/day of Xylitol |
| PDXh (Polydextrose) - | 25g/day of h-Polydextrose |

Volunteers were required to consume one portion (200g) of yoghurt on each of the 10 days. Therefore, energy content of the yoghurts varied between the four yoghurt formulations while weight was held constant. The energy and nutrient content of each yoghurt formulation is presented in the following Table.

**TABLE I. ENERGY AND NUTRIENT VALUES FOR THE YOGHURT FORMULATIONS, VALUES PER 200g.**

| | Control | Xylitol | PDXh | XylPDXh |
|---|---|---|---|---|
| Energy - kcal | 204 | 164 | 130 | 146 |
| Protein - q | 8.16 | 8.16 | 8.16 | 8.16 |
| Carbohydrate - g | 35.6 | 35.6 | 35.6 | 35.6 |
| Fat - g | 3.3 | 3.3 | 3.3 | 3.3 |

### Procedure

### Test days 1 and 10

Subjects consumed the yoghurts for 10 consecutive days. However, days 2-9 were free-living during which time they were not required to visit the human appetite research unit (HARU), where the studies described hereinbelow were performed. On days 1 and 10 (test days), subjects were required to visit the HARU for a fixed breakfast at 8:30 a.m. and an *ad libitum* test lunch. The energy and nutrient content of the breakfast, was fixed for each of the conditions. Immediately after breakfast subjects were free to leave the unit and instructed to consume the yoghurt at 11:00am and not to consume any other food or drink during the breakfast-lunch interval. At 12:30 subjects returned to the unit for the *ad libitum* test lunch and were instructed to eat until a comfortable level of fullness. The food was weighed immediately before and after the test lunch to determine the energy and nutrient of food consumed. The test lunch was mixed composition (31% Fat, 53% Carbohydrate and 16% Protein) and consisted of sandwiches, crisps and fruit. Immediately after the test lunch subjects were provided with their yoghurts for days 2-9 and were instructed to return at 8:30 on day 10.

In addition, on days 1 and 10, subjective ratings of motivation to eat were periodically monitored using the Electronic Appetite Ratings System (EARS). ' This is an electronic method of using visual analogue scales (VAS) to measure subjective states. The EARS procedure has been described in De Largy, H.J., Lawton, C.L., Smith, F.C., King, N.A. and Blundell, J.Z. (1996); Int. J. Obes., 20, 104S and King, N.A., Lluch, A., Stubbs, R.J. and Blundell, J.E. (1997), Eur. J. Clin. Nutr., 42, 956-965, the contents of which are incorporated herein by reference. Subjects typically completed the subjective ratings immediately before and after food consumption (i.e. breakfast, yoghurt and test lunch) and every hour during the meal intervals.

On days 1 and 10, volunteers were provided with an *ad libitum* lunch 1.5 hours following consumption of the yoghurt pre-load.

### RESULTS

The results are as follows. There were substantial differences in energy intake (EI) between the four conditions, with intake after the control yoghurt always being the largest. Analysis of variance (ANOVA) revealed that there was a weak marginally significant effect of yoghurt condition on energy intake (F(3,14)=2.84, p=0.092). There was no significant effect of day or gender on energy intake. However, although the ANOVA indicated that there was no significant main effect of yoghurt type on average energy intake, paired comparison showed that the difference in average energy intake between the control) and Xyl conditions was statistically significant (t=2.92, d.f.=14, p<0.05). The suppression induced by the Xyl, PDXh and XylPDXh yoghurts (compared with the control) were 11.9%, 9.9% and 7.2% respectively. See Table II.

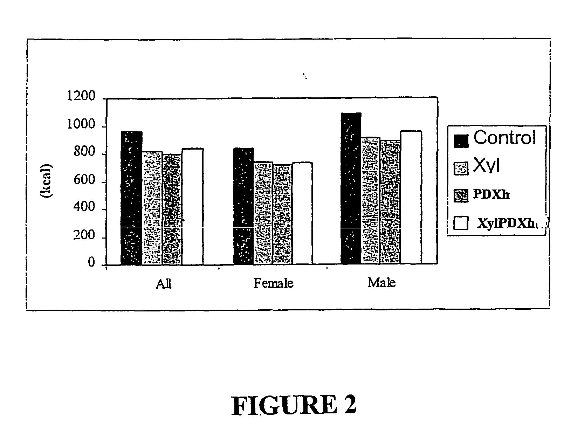

Since the yoghurt pre-loads varied in energy content, the analysis was repeated after combining the energy contents of the yoghurt pre-loads with the test meal intakes. When the energy content of the yoghurt pre-loads was accounted for, the suppression of energy intake at the test meal was greater (Xyl -14.8%; PDXh - 16.8% ; XylPDXh - 13%). See Table III and Figure 2. ANOVA revealed a highly significant difference in combined energy intake between the four yoghurts (F(3,11)=7.95, p<0.005). There was a significant difference between each experimental yoghurt and the control yoghurt. Post-hoc paired comparisons revealed that the combined intakes of Xyl, PDXh and XylPDXh conditions were significantly lower compared with the control (lowest t=2.95, d.f.=14, p<0.05). Therefore, when the energy content of the pre-load was accounted for, the suppression of intake induced by the experimental yoghurts was highly significant.

The h-polydextrose and the h-polydextrosexylitol significantly reduced the hunger of the subjects.

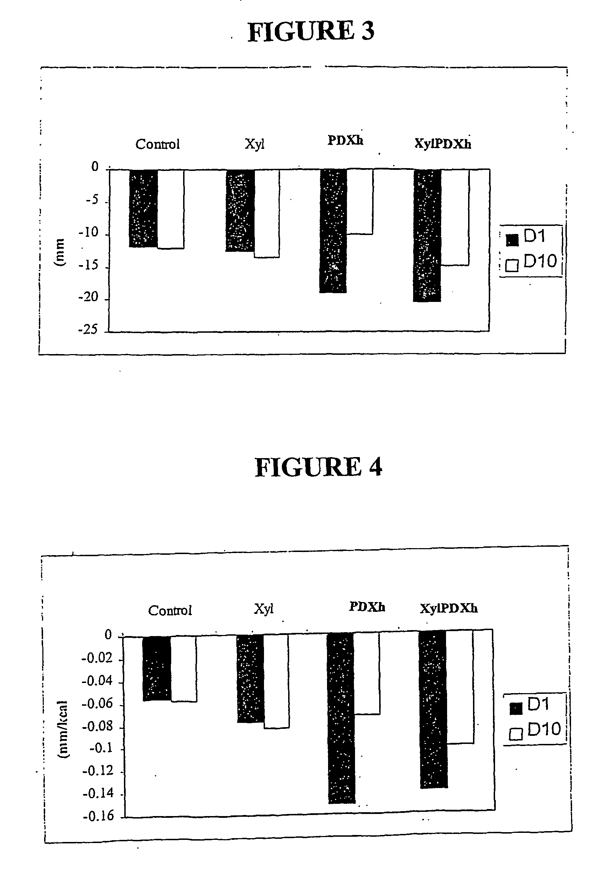

Since volunteers rated their subjective states immediately before and immediately after consumption of the yoghurt on test days 1 and 10, it was possible to determine the satiating effect (i.e. suppression of hunger, or increase in fullness) of the four yoghurts. The XylPDXh and PDXh yoghurts showed the strongest suppression of hunger on day 1, as shown in Figure 3.

However, it is more appropriate to calculate a 'relative' suppression by expressing the suppression of hunger as a function of the energy content of the yoghurt pre-load - to account for the differential energy content. Similar to the energy intakes, when the energy differential between the yoghurts was accounted for, the suppression of hunger was greater.

When the caloric values of the test yoghurts are factored in, ANOVA revealed that there was a significant effect of yoghurt condition on hunger (F(3,11)=7.74, p<0.01. Post-hoc t-tests revealed that the increase in fullness and hunger induced by the PDXh (t=2.49, d.f.=14, p<0.05; t=2.66, d.f..=14, p<0.05) respectively and XylPDXh (t=3.28, d.f.=14, p<0.05; t=3.11, d.f.=14, p<0.01 respectively) yoghurts was significantly greater compared with the control yoghurt, as shown in Figure 4.

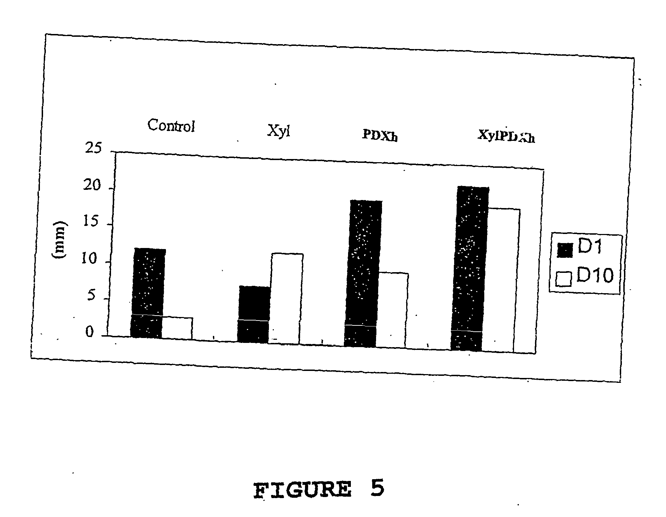

Figure 5 depicts the increase in fullness induced by the yoghurts. As clearly shown, both h-polydextrose and the h-polydextrose/xylitol were the most effective in inducing fullness in the subjects.

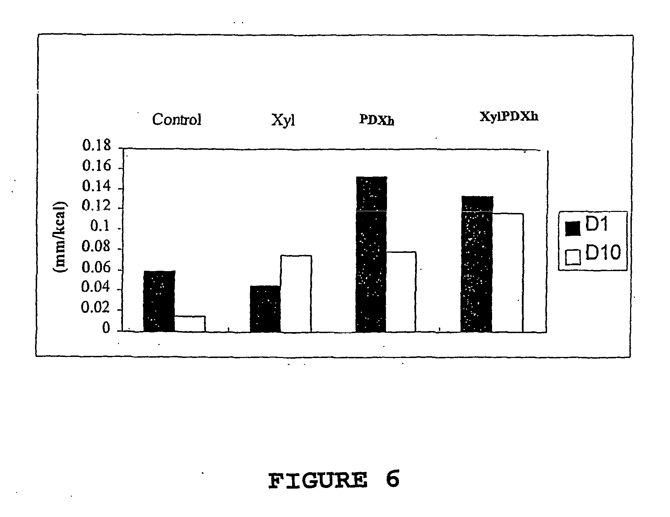

Figure 6 shows the relative increase on fullness (factoring in yoghurt calorie content) induced by the consumption of the yoghurt pre-loads on days 1 and 10. As clearly shown by the data, both h-polydextrose and h-polydextrose/xylitol mixture provided the most enhanced fullness in subject.

The results of this study have demonstrated that Polydextrose and polydextrose/xylitol mixture have the potential to reduce hunger and suppress later food consumption when consumed in a test yoghurt (pre-load) 90 minutes before a test meal.

The data also show another embodiment of the present invention, i.e., the effect of hydrogenated polydextrose and the synergistic effect of hydrogenated polydextrose in combination with xylitol. These effects are apparent from Figures 3 and 4 and 5 and 6 which show that suppression of hunger and enhancement of fullness diminish considerably for h-polydextrose, after consumption of the test yoghurts for 10 days. The synergy of the mixture shown is via its improved ability to control hunger after ten days, compared with the h-polydextrose alone. Furthermore, the combination of polydextrose with xylitol helps to maintain the suppression of hunger, compared with polydextrose alone.

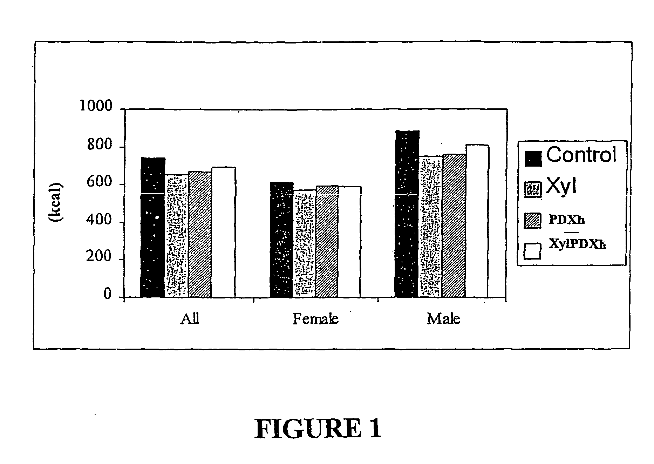

Although only the xylitol condition resulted in a significant reduction in energy intake at the test meal (see Table II and Figure 1), all of the test conditions reduced the combined energy intake when the data was standardized to take account of the preload. (See Table III and Figure 2.)

**TABLE II. SUPPRESSION (%) IN ENERGY INTAKE INDUCED BY THE THREE EXPERIMENTAL YOGHURTS COMPARED WITH THE CONTROL YOGHURT**

| | **All** | **Females** | **Males** |
|---|---|---|---|
| **Xyl** | 11.9 | 6.4 | 15.3 |
| **PDXh** | 9.9 | 3.5 | 14.2 |
| **XylPDXh** | 7.2 | 4.1 | 8.7 |

Since the yoghurt preloads varied in energy content, to standardize the results, the above analysis was repeated after combining the energy contents of the yoghurt pre-loads with the test meal intake. The data is depicted in Table III.

**TABLE III. SUPPRESSION (%) IN COMBINED PRELOAD PLUS TEST MEAL ENERGY INTAKE INDUCED BY THE THREE EXPERIMENTAL YOGHURTS COMPARED WITH THE CONTROL YOGHURT.**

| | **All** | **Females** | **Males** |
|---|---|---|---|
| **Xyl** | 14.8 | 11.6 | 16.1 |
| **PDXh** | 16.8 | 13.5 | 18.3 |
| **XylPDXh** | 10.9 | 9.6 | 10.6 |

As clearly shown by the data in Tables II and III and depicted in the graphs in Figures 1 and 2, when the caloric contribution of both the preload and the test meal are taken into account, h-polydextrose and the h-polydextrose/xylitol conditions give a significant calorie reduction relative to the control, as did xylitol alone. Thus, another role of the h-polydextrose is to suppress calorie intake via its inherently lower calorie value. A combination of xylitol and h-polydextrose acts synergistically by taking advantage of both effects.

These data illustrate another embodiment of the present invention. This other embodiment of the present invention is directed to the synergistic combination of h-polydextrose and xylitol in effecting appetite suppression and/or reducing caloric intake. The h-polydextrose and the xylitol are present in a synergistic effective amount to suppress hunger and/or calorie intake. In other words, as shown by the data, the present inventors have found that when the xylitol and h-polydextrose were used in synergistic effective amounts, the combination resulted in greater suppression of hunger and enhancement of fullness.

As indicated hereinabove, the xylitol and h-polydextrose are administered in synergistic effective amounts. The total amount of the xylitol and polydextrose are in the amounts given for the satiety agents described hereinabove. However, within this range, the h-polydextrose and the xylitol are preferably present in weight ratios ranging from about 1:10 to about 10:1 and more preferably from about 1:5 to about 5:1 and most preferably at about 1:1.

Thus, the present invention is directed to the synergistic effect of polydextrose, as defined herein, including hydrogenated polydextrose, and xylitol in suppressing the appetite of the animal e.g., mammal, and/or in reducing food intake. Both are present in synergistic effective amounts. The preferred amounts are within the ranges given hereinabove with respect to h-polydextrose and xylitol.

A further embodiment of the present invention is directed to the synergistic effect in suppressing the appetite of the animal, e.g., mammal and/or in reducing food intake when a synergistic effective amount of xylitol and the mixture of polydextrose and hydrogenated polydextrose is administered to the animal. It is preferred that the weight ratio of the mixture of polydextrose and hydrogenated polydextrose to xylitol ranges from about 1:10 to about 10:1 and more preferably from about 1:5 to about 5:1 and most preferably at about 1:1.

Besides xylitol, the polydextrose and hydrogenated polydextrose, alone or in combination exhibit a synergistic effect with other sugar alcohols, as defined hereinabove.

Besides polydextrose, other sugar polymers as defined herein can also be used as satiety agents. These sugar polymers are non-toxic and safe.

In an embodiment of the present invention, the sugar polymer may be used hereinabove in lieu of the polydextrose described hereinabove and administered to the animal to control the appetite and/or food or calorie intake thereof. Thus, in another embodiment of the present invention, the sugar polymers are also useful as satiety agents. They are administered to animals, including mammals, e.g., and humans, in appetite suppressing effective amounts as described hereinabove. The preferred amounts are the amounts described hereinabove for polydextrose.

A type of sugar polymer, e.g., hydrogenated sugar polymer, may also be utilized as a satiety agent. The processes for hydrogenating polydextrose, which is known in the art, is also applicable to hydrogenating the sugar polymers prepared in accordance with the present invention.

Furthermore, the sugar polymer, including the hydrogenated sugar polymer, either alone or in combination, act with xylitol or other sugar alcohol in synergism to control the appetite of the animal and/or provide fullness. They are present in synergistic effective amounts as described hereinabove. The preferred ratios described hereinabove with respect to polydextrose and xylitol are also applicable to the sugar polymer (including hydrogenated sugar polymer or mixture of non-hydrogenated sugar polymer and hydrogenated sugar polymer) and polyol.

### EXAMPLE 2

The procedure of Example 1 was repeated except that the yoghurt containing the xylitol, h-polydextrose and the 1:1 mixture by weight of xylitol and h-polydextrose were given at breakfast at 8:30 am, i.e., four hours prior to the test meal at 12:30 p.m.

### RESULTS

In general, there was an increase in satiety observed at the test lunch 4 hours following consumption of the experimental yoghurts, i.e., yogurts containing either xylitol, h-polydextrose or 1:1 mixture of xylitol and h-polydextrose, relative to the control. The suppressive effects were not diminished by repeated exposure over the ten days. However, the suppressive effect overall with respect to all volunteers was greater (7-15% reduction) when the preload was taken 60-90 minutes before the test meal than when it was taken together with breakfast 4 hours before the test meal (~3-4% reduction). Nevertheless, when the caloric contribution of the yoghurt was accounted for , the suppressive effect for all volunteers was (5-8%).

Unless indicated to the contrary, the percentages are weight percentages. Moreover, the weights provided are the dry weights, i.e., excluding the weight of the carrier that may be present.

As used herein, the term "sugar alcohol" and "polyol" are used interchangeably.

Moreover, the singular denotes the plural and vice versa.

The above preferred embodiments and examples are given to illustrate the scope and spirit of the present invention. These embodiments and examples will make apparent to those skilled in the art other embodiments and examples. These other embodiments and examples are within the contemplation of the present invention.

Therefore, the present invention should be limited only by the appended claims.

## Claims

1. Use of an enzyme resistant sugar polymer, wherein the enzyme resistant sugar polymer is polydextrose, galactooligosaccharide, pyrodextrin, inulin, hydrolyzed guar gum or a sugar polymer, wherein the sugar is polymerized by polycondensation in the presence of an acid, and wherein the sugar is a mono,-di-, or oligosaccharide and includes glyceraldehyde, erythrose, threose, ribose, arabinose, xylose, lyxose, allose, altrose, glucose, mannose, idose, galactose, talose, erythrulose, ribulose, xylulose, psicose, fructose, sorbose, tagatose, maltose, lactose, sucrose, trehalose, isomaltose, isomaltulose, fructo-oligosaccharide or maltotriose, for manufacturing an appetite suppressing medicine, which comprises a food intake suppressing amount of said enzyme resistant sugar polymer.

2. Non-therapeutic use of an enzyme resistant sugar polymer, wherein the enzyme resistant sugar polymer is polydextrose, galactooligosaccharide, pyrodextrin, inulin, hydrolyzed guar gum or a sugar polymer, wherein the sugar is polymerized by polycondensation in the presence of an acid, and wherein the sugar is a mono-, di-, or oligosaccharide and includes glyceraldehyde, erythrose, threose, arabinose, xylose, lyxose, allose, altrose, mannose, idose, galactose, talose, erythrulose, ribulose, xylulose, psicose, fructose, sorbose, tagatose, lactose, maltose, sucrose, trehalose, isomaltose, isomaltulose, fructo-oligosaccharide or maltotriose, for suppressing the appetite of a mammal.

3. The use according to claim 1 or claim2, wherein the enzyme resistant sugar polymer is polydextrose, galactooligosaccharide, pyrodextrin, inulin, hydrolyzed guar gum.

4. The use according to claim 1 or claim 2, wherein said enzyme resistant sugar polymer is non-hydrogenated sugar polymer, hydrogenated sugar polymer, non-hydrogenated or hydrogenated sugar polymer, which has been subjected to purification, or a mixture thereof.

5. The use according to claim 1 or claim 2, wherein said enzyme resistant sugar polymer is hydrogenated sugar polymer.

6. The use according to claim 1 or claim 2, wherein said enzyme resistant sugar polymer is polydextrose, hydrogenated polydextrose, or purified polydextrose.

7. The use according to claim 1 or claim 2, wherein the enzyme resistant sugar polymer is polydextrose.

8. The use according to claim 1 or claim 2, wherein the enzyme resistant sugar polymer is hydrogenated polydextrose.

9. The use according to claim 1 or claim 2, wherein the enzyme resistant sugar polymer is purified polydextrose.

10. The use according to claim 1 or claim 2, wherein the enzyme resistant sugar polymer is polydextrose and wherein the food intake suppressing amount of said enzyme resistant sugar polymer ranges from about 1 g to about 50 g per day, preferably from about 15 g to about 30 g per day.

11. The use according to any of claims 1-10, wherein a sugar alcohol or a polyol is additionally present in synergistically effective amounts.

12. The use according to claim 11, wherein the sugar alcohol is xylitol.

13. The use according to claim 11, wherein said sugar polymer and sugar alcohol are present in an amount having a synergistic effect on the suppression of food intake.

14. The use according to claim 13, wherein the sugar alcohol is xylitol and the sugar polymer is polydextrose and the weight ratio of xylitol to polydextrose ranges from about 1:10 to about 10:1, preferably from about 1:5 to about 5:1, most preferably being about 1:1.

15. The use according to any of the claims 1 to 14, wherein the enzyme resistant sugar polymer and optional sugar alcohol polyol composition is provided in food.

16. The use according to any of claims 1 to 15, wherein the enzyme resistant sugar polymer and the optional sugar alcohol polyol composition is provided during a scheduled meal or snack.

17. The use according to any of claims 1 to 15, wherein the sugar polymer and the optional sugar alcohol polyol composition is provided in a time period ranging from just prior to a scheduled meal or snack up to about four hours prior to a scheduled meal or snack.

18. The use according to any of claims 1 to 15, wherein a taste-making agent is additionally present.

19. The use according to any of claims 1 to 15, wherein a sweetener is additionally present.

20. The use according to claim 19, wherein the sweetener is aspartame, cyclamate, saccharin, acesulfame, neohesperidin dihydrochalcone, sucralose, alitame, stevia sweetener, glycyrrhisin, thaumatin or mixtures thereof.

21. The use according to claim 19, wherein the sweetener is aspartame.

22. The use according to claim 19, wherein the sweetener is a dipeptide sweetener.

23. The use according to any of the claims 1 to 15, wherein a nonpolyol appetite suppressant is additionally present.

## Patentansprüche

1. Verwendung eines enzymresistenten Zuckerpolymers, wobei das enzymresistente Zuckerpolymer Polydextrose, Galactooligosaccharid, Pyrodextrin, Inulin, hydrolysiertes Guargummi oder ein Zuckerpolymer ist, wobei der Zucker durch Polykondensation in Gegenwart einer Säure polymerisiert ist und wobei der Zucker ein Mono-, Di- oder Oligosaccharid ist und Glyceraldehyd, Erythrose, Threose, Ribose, Arabinose, Xylose, Lyxose, Allose, Altrose, Glucose, Mannose, Idose, Galactose, Talose, Erythrulose, Ribulose, Xylulose, Psicose, Fructose, Sorbose, Tagatose, Maltose, Lactose, Saccharose, Trehalose, Isomaltose, Isomaltulose, Fructo-Oligosaccharid oder Maltotriose beinhaltet, zur Herstellung einer appetitzügelnden Medizin, umfassend eine Nahrungsmittelaufnahme unterdrückende Menge des enzymresistenten Zuckerpolymers.

2. Nicht-therapeutische Verwendung eines enzymresistenten Zuckerpolymers, wobei das enzymresistente Zuckerpolymer Polydextrose, Galactooligosaccharid, Pyrodextrin, Inulin, hydrolysiertes Guargummi oder ein Zuckerpolymer ist, wobei der Zucker durch Polykondensation in Gegenwart einer Säure polymerisiert ist und wobei der Zucker ein Mono-, Di- oder Oligosaccharid ist und Glyceraldehyd, Erythrose, Threose, Arabinose, Xylose, Lyxose, Allose, Altrose, Mannose, Idose, Galactose, Talose, Erythrulose, Ribulose, Xylulose, Psicose, Fructose, Sorbose, Tagatose, Lactose, Maltose, Saccharose, Trehalose, Isomaltose, Isomaltulose, Fructo-Oligosaccharid oder Maltotriose beinhaltet, zur Unterdrückung des Appetits eines Säugers.

3. Verwendung gemäß Anspruch 1 oder 2, wobei das enzymresistente Zuckerpolymer Polydextrose, Galactooligosaccharid, Pyrodextrin, Inulin oder hydrolysiertes Guargummi ist.

4. Verwendung gemäß Anspruch 1 oder 2, wobei das enzymresistente Zuckerpolymer ein nicht hydriertes Zuckerpolymer, hydriertes Zuckerpolymer, nichthydriertes oder hydriertes Zuckerpolymer ist, das einer Reinigung unterworfen wurde, oder eine Mischung davon.

5. Verwendung gemäß Anspruch 1 oder 2, wobei das enzymresistente Zuckerpolymer ein hydriertes Zuckerpolymer ist.

6. Verwendung gemäß Anspruch 1 oder 2, wobei das enzymresistente Zuckerpolymer Polydextrose, hydrierte Polydextrose oder gereinigte Polydextrose ist.

7. Verwendung gemäß Anspruch 1 oder 2, wobei das enzymresistente Zuckerpolymer Polydextrose ist.

8. Verwendung gemäß Anspruch 1 oder 2, wobei das enzymresistente Zuckerpolymer hydrierte Polydextrose ist.

9. Verwendung gemäß Anspruch 1 oder 2, wobei das enzymresistente Zuckerpolymer gereinigte Polydextrose ist.

10. Verwendung gemäß Anspruch 1 oder 2, wobei das enzymresistente Zuckerpolymer Polydextrose ist und wobei die die Nahrungsmittelaufnahme unterdrückende Menge des enzymresistenten Zuckerpolymers zwischen ungefähr 1 g bis ungefähr 50 g pro Tag, vorzugsweise zwischen ungefähr 15 g und ungefähr 30 g pro Tag, liegt.

11. Verwendung gemäß einem der Ansprüche 1 bis 10, wobei ein Zuckeralkohol oder eine Polyol zusätzlich in synergistisch wirksamen Mengen vorliegt.

12. Verwendung gemäß Anspruch 11, wobei der Zuckeralkohol Xylit ist.

13. Verwendung gemäß Anspruch 11, wobei das Zuckerpolymer und der Zuckeralkohol in einer Menge mit einer synergistischen Wirkung auf die Unterdrückung der Nahrungsmittelaufnahme vorliegen.

14. Verwendung gemäß Anspruch 13, wobei der Zuckeralkohol Xylit ist und das Zuckerpolymer Polydextrose, und wobei das Gewichtsverhältnis von Xylit zu Polydextrose in einem Bereich von ungefähr 1:10 bis ungefähr 10:1, vorzugsweise ungefähr 1:5 bis ungefähr 5:1, noch bevorzugter bei ungefähr 1:1 liegt.

15. Verwendung gemäß einem der Ansprüche 1 bis 14, wobei das enzymresistente Zuckerpolymer und die optionale Zuckeralkoholpolyolzusammensetzung in einem Nahrungsmittel bereitgestellt wird.

16. Verwendung gemäß einem der Ansprüche 1 bis 15, wobei das enzymresistente Zuckerpolymer und die optionale Zuckeralkoholpolyolzusammensetzung während einer festgelegten Mahlzeit oder einem Snack bereitgestellt werden.

17. Verwendung gemäß einem der Ansprüche 1 bis 15, wobei das Zuckerpolymer und die optionale Zuckeralkoholpolyolzusammensetzung in einer Zeitspanne von direkt vor einer festgelegten Mahlzeit oder einem Snack bis zu ungefähr vier Stunden vor der festgelegten Mahlzeit oder dem Snack bereitgestellt wird.

18. Verwendung gemäß einem der Ansprüche 1 bis 15, wobei zusätzlich ein Geschmacksmaskierungsmittel vorliegt.

19. Verwendung gemäß einem der Ansprüche 1 bis 15, wobei zusätzlich ein Süßmittel vorliegt.

20. Verwendung gemäß Anspruch 19, wobei das Süßmittel Aspartam, Cyclamat, Saccharin, Acesulfam, Neohesperidindihydrochalcon, Sucralose, Alitam, Stevia-Süßmittel, Glycyrrhizin, Thaumatin oder Mischungen davon ist.

21. Verwendung gemäß Anspruch 19, wobei das Süßmittel Aspartam ist.

22. Verwendung gemäß Anspruch 19, wobei das Süßmittel ein Dipeptid-Süßmittel ist.

23. Verwendung gemäß einem der Ansprüche 1 bis 15, wobei zusätzlich ein Nicht-Polyol-Appetitunterdrückungsmittel vorliegt.

## Revendications

1. Utilisation d'un polymère de sucre résistant aux enzymes, dans laquelle le polymère de sucre résistant aux enzymes est le polydextrose, un galactooligosaccharide, la pyrodextrine, l'inuline, la gomme de guar hydrolysée ou un polymère de sucre, dans laquelle le sucre est polymérisé par polycondensation en présence d'un acide, et dans laquelle le sucre est un mono-, di-, ou oligosaccharide et comprend le glycéraldéhyde, l'érythrose, le thréose, le ribose, l'arabinose, le xylose, le lyxose, l'allose, l'altrose, le glucose, le mannose, l'idose, le galactose, le talose, l'érythrulose, le ribulose, le xylulose, le psicose, le fructose, le sorbose, le tagatose, le maltose, le lactose, le saccharose, le tréhalose, l'isomaltose, l'isomaltulose, le fructo-oligosaccharide ou le maltotriose, pour la fabrication d'un médicament supprimant l'appétit qui comprend une quantité supprimant l'absorption de nourriture dudit polymère de sucre résistant aux enzymes.

2. Utilisation non thérapeutique d'un polymère de sucre résistant aux enzymes, dans laquelle le polymère de sucre résistant aux enzymes est le polydextrose, un galactooligosaccharide, la pyrodextrine, l'inuline, la gomme de guar hydrolysée ou un polymère de sucre, dans laquelle le sucre est polymérisé par polycondensation en présence d'un acide, et dans laquelle le sucre est un mono- , di-, ou oligosaccharide et comprend le glycéraldéhyde, l'érythrose, le thréose, le ribose, l'arabinose, le xylose, le lyxose, l'allose, l'altrose, le mannose, l'idose, le galactose, le talose, l'érythrulose, le ribulose, le xylulose, le psicose, le fructose, le sorbose, le tagatose, le lactose, le maltose, le saccharose, le tréhalose, l'isomaltose, l'isomaltulose, le fructo-oligosaccharide ou le maltotriose, pour supprimer l'appétit d'un mammifère.

3. Utilisation selon la revendication 1 ou la revendication 2, dans laquelle le polymère de sucre résistant aux enzymes est le polydextrose, un galactooligosaccharide, la pyrodextrine, l'inuline, la gomme de guar hydrolysée.

4. Utilisation selon la revendication 1 ou la revendication 2, dans laquelle le polymère de sucre résistant aux enzymes est un polymère de sucre non hydrogéné, un polymère de sucre hydrogéné, un polymère de sucre non hydrogéné ou hydrogéné, qui a été soumis à une purification, ou un mélange de ceux-ci.

5. Utilisation selon la revendication 1 ou la revendication 2, dans laquelle ledit polymère de sucre résistant aux enzymes est un polymère de sucre hydrogéné.

6. Utilisation selon la revendication 1 ou la revendication 2, dans laquelle ledit polymère de sucre résistant aux enzymes est le polydextrose, le polydextrose hydrogéné ou le polydextrose purifié.

7. Utilisation selon la revendication 1 ou la revendication 2, dans laquelle le polymère de sucre résistant aux enzymes est le polydextrose.

8. Utilisation selon la revendication 1 ou la revendication 2, dans laquelle le polymère de sucre résistant aux enzymes est le polydextrose hydrogéné.

9. Utilisation selon la revendication 1 ou la revendication 2, dans laquelle le polymère de sucre résistant aux enzymes est le polydextrose purifié.

10. Utilisation selon la revendication 1 ou la revendication 2, dans laquelle le polymère de sucre résistant aux enzymes est le polydextrose et dans laquelle la quantité supprimant l'absorption de nourriture dudit polymère de sucre résistant aux enzymes est dans la gamme d'environ 1 g à environ 50 g par jour, de préférence d'environ 15 g à environ 30 g par jour.

11. Utilisation selon l'une quelconque des revendications 1 à 10, dans laquelle un alcool de sucre ou un polyol est en outre présent en des quantités synergiquement efficaces.

12. Utilisation selon la revendication 11, dans laquelle l'alcool de sucre est le xylitol.

13. Utilisation selon la revendication 11, dans laquelle ledit polymère de sucre et ledit alcool de sucre sont présents en une quantité ayant un effet synergique sur la suppression de l'absorption de nourriture.

14. Utilisation selon la revendication 13, dans laquelle l'alcool de sucre est le xylitol et le polymère de sucre est le polydextrose et le rapport en poids du xylitol sur le polydextrose est dans la gamme d'environ 1: 10 à environ 10: 1, de préférence d'environ 1: 5 à environ 5: 1, de manière préférée entre toutes d'environ 1: 1.

15. Utilisation selon l'une quelconque des revendications 1 à 14, dans laquelle le polymère de sucre résistant aux enzymes et la composition polyol d'alcool de sucre facultative sont fournis dans la nourriture.

16. Utilisation selon l'une quelconque des revendications 1 à 15, dans laquelle le polymère de sucre résistant aux enzymes et la composition polyol d*'*alcool de sucre facultative sont fournis pendant un repas ou un en-cas régulier.

17. Utilisation selon l'une quelconque des revendications 1 à 15, dans laquelle le polymère de sucre et la composition polyol d'alcool de sucre facultative sont fournis sur une période allant de juste avant un repas ou un en-cas régulier jusqu' à environ 4 heures avant un repas ou un en-cas régulier.

18. Utilisation selon l'une quelconque des revendications 1 à 15, dans laquelle un agent exhausteur de goût est en outre présent.

19. Utilisation selon l'une quelconque des revendications 1 à 15, dans laquelle un édulcorant est en outre présent.

20. Utilisation selon la revendication 19, dans laquelle l'édulcorant est l'aspartame, le cyclamate, la saccharine, l'acésulfame, la néohespéridine, la dihydrochalcone, le sucralose, l'alitame, un édulcorant à base de stevia, la glycyrrhisine, la thaumatine ou les mélanges de ceux-ci.

21. Utilisation selon la revendication 19, dans laquelle l'édulcorant est l'aspartame.

22. Utilisation selon la revendication 19, dans laquelle l'édulcorant est un édulcorant dipeptide.

23. Utilisation selon l'une quelconque des revendications 1 à 15, dans laquelle un suppresseur de l'appétit non polyol est en outre présent.
